# EUROPEAN PATENT APPLICATION

(11) **EP 4 631 541 A1**
(43) Date of publication of application: **15.10.2025**
(21) Application number: 24169336.5
(22) Date of filing: 09.04.2024
(51) Int. Cl.: A61L 31/04, A61L 31/12, A61L 31/14, A61L 31/16

(54) **A MEDICAL DEVICE, A SET, AND A METHOD OF MANUFACTURING A MEDICAL DEVICE**

(71) Applicant: ZuriMED Technologies AG, 8008 Zürich (CH)
(72) Inventor: SCHLEGEL, Cornelius, 8008 Zürich (CH); MARCELO, Catherine, 8008 Zürich (CH); BANZET, Pol, 8008 Zürich (CH); EGLAUF, Janick, 8008 Zürich (CH); BACHMANN, Elias, 8049 Zürich (CH); LI, Xiang, 8610 Uster (CH)
(74) Representative: Vossius & Partner Patentanwälte Rechtsanwälte mbB

(57) **Abstract**

The present disclosure relates to an implantable medical device (1) for tissue repair or closure. The device comprises a patch (2) and a hydrogelating material (3). The patch comprises a felt for tissue repair or closure. The hydrogelating material is configured to retain an active ingredient. The hydrogelating material may be biodegradable with a predetermined biodegradation rate to release the active ingredient. The hydrogelating material may be configured to release the active ingredient in response to a trigger.

## Description

The present invention relates to a medical device, a set, and a method of manufacturing a medical device

The present applicant developed a novel surgical felting device, which offers biomechanical benefits in attaching an implant to soft tissue compared to traditional suturing methods. This device features a surgical instrument equipped with a surgical felting needle that repeatedly passes through a surgical felt of the implant and into the tissue. By embedding strands of the felt within the tissue, it establishes a robust and evenly distributed mechanical bond between the felt and the soft tissue.

A surgical procedure during which the surgical device is used to attach an implant to soft tissue might necessitate additional measures to enhance compatibility, healing, or other functions. This could involve administering drugs before or after felting, which can be cumbersome and may result in drug distribution to unintended areas. The surgical felt has a fibrous and porous structure. Due to these properties, it can be used as a vehicle to carry a drug or bioactive factors for a drug release after attaching the medical felt into tissue, as for example described in WO 2023/232780. The felt's fibers can contain the drug. For instance, the fibers might be composed of or include the drug. The drug could be adsorbed or absorbed by the fibers.

It is also possible that the drug is solid and forms an integral part of the fiber. Consequently, this mechanism allows for direct delivery of the drug to soft tissues, achieving targeted drug delivery combined with a secure mechanical anchoring. The drug may be arranged between and/or around the filaments of the fibers, e.g. soaking, spraying, or powdering the drug on the fibers.

In most clinical cases involving soft-tissue repair or closure, a surgical felting device is used to mechanically fix the defect by felting a patch to soft tissue. However, a biological factor or drug to improve healing, prevent infections, or provide pain relief is often needed but not yet available for most surgical patches or meshes. Some porous patches, as described in the patent application WO 2023/232780, may carry drugs or bioactive factors to a certain extent. Still, limitations in the micromorphology of the porous structure and the surface properties of the materials and fibers restrict precise control over dosage and release profile, limiting their clinical application, in particular when the patch is felted to soft tissue.

It is the aim to overcome the above disadvantages of the prior art. In particular, it is the problem to provide a medical device that can be felted and releases a drug according to a pre-determined release profile. The problem is solved by the features of the independent claims.

A first aspect of the present disclosure relates to an implantable medical device for tissue repair or closure. The device comprises a patch and a hydrogelating material. The patch comprises a felt for tissue repair or closure. The hydrogelating material is configured to retain an active ingredient. The hydrogelating material may be biodegradable with a predetermined biodegradation rate to release the active ingredient. The hydrogelating material may be configured to release the active ingredient in response to a trigger. In some examples, the hydrogelating material degrades in response to the trigger.

The disclosed patch provides a targeted drug delivery in feltable patches. The combination of a hydrogel with a felt results in an implant that can provide mechanical stability with a well distributed anchoring and targeted and predetermined release of an active ingredient precisely at the target site. The disclosed device is compatible with the soft tissue felting techniques developed by the present applicant while also taking advantage of a release of active ingredients using a hydrogel. Hydrogels comprising fibers with a high yield strength (100 kPa or more) may be particularly suitable for felting.

The medical device may be an implant. The implant may be configured to remain in a human (or animal body) after surgery. The medical device may have the shape of a sheet. The device may be flexible, i.e. bendable, to conform to the shape of soft tissue.

The hydrogelating material may include a network of cross-linked fibers. The hydrogelating material may retain water. The hydrogelating material may comprise polymer. The hydrogelating material may be able to retain at least 10% its own weight in water. Preferably, the hydrogel has a swelling ratio of at least one, two, five, or ten times its own weight. In some examples, the hydrogel has a swelling ratio of at least 12, 15, 18 or 20 times its own weight. The swelling ratio may be calculated as the weight of the absorbed water divided by the weight of the hydrogelating material prior to swelling.

Preferably, the device is configured to be felted. The felt may be configured to be felted, if the fibers have a tenacity as described below. However, more generally, the felt may be configured to be felted, if the fibers of the felt (or the hydrogelating material or hydrogel) can be pushed or pulled by a felting needle into soft tissue. In particular the felt may be feltable using a reciprocally moving felting needle as will be described in one example below.

Preferably, the hydrogelating material comprises or is made of a natural polymer or a synthetic polymer or a combination thereof.

Preferably, the natural polymer comprises or is at least one of: cellulose, in particular bacterial cellulose, alginate, hyaluronic acid, HA, gelatine, agar, agarose, starch, chitosan, and fibrinogen. These polymers are particularly suitable for a desired release profile. One particular example is carboxymethyl cellulose, CMC.

Preferably, the synthetic polymer is at least one of: an acrylamide, an acrylic acid, a salt of an acrylic acid, and 2-Hydroxyethyl Methacrylate, PHEMA. These polymers are particularly suitable for a desired release profile.

Preferably, the hydrogelating material forms a hydrogel and may be partially swollen with water. A hydrogel may be a gel in which the swelling agent is water. A hydrogelating material as mentioned herein may refer to the capability of the hydrogelating material to hold water without necessarily holding water. In some case a user (e.g. a clinical user such as a doctor or nurse) may swell the hydrogelating material to form a hydrogel, while in other cases the hydrogelating material may be swollen while manufacturing the disclosed device at a manufacturer's site. A hydrogelating material may mean that the material is already cross-linked but is not fully swollen.

Swelling the hydrogelating material to a hydrogel may include loading the hydrogel with the active ingredient.

The active ingredient may be hydrophilic. The active ingredient may be water-soluble. The active ingredient may be a small molecule drug (e.g., ≤ 1000 daltons). Small molecule drugs may diffuse more easily into the hydrogel. The active ingredient may chemically match the hydrogel to avoid degradation or altering of the hydrogel structure caused by drug molecules. The active ingredient may have a matching charge to the hydrogel. E.g., an anionic hydrogel may be used in combination with positively charged active ingredients and/or a cationic hydrogel may be used in combination with negatively charged active ingredients (anions).

Preferably, the hydrogelating material forms a hydrogel and the hydrogel is fully swollen with water. The hydrogel may contain an active ingredient that is dissolved or otherwise held (e.g., suspended and/or emulsified) within the water.

Preferably, the active ingredient is water-based and in particular dissolved in the water held by the hydrogel. Preferably, the active ingredient is dispersed, suspended, and/or emulsified in the water held by the hydrogel. Thereby, the active ingredient may be loaded in the disclosed device.

Preferably, the active ingredient is at least one of: a drug, a bioactive component, and a biologic.

Preferably, the active ingredient comprises or is at least one of: a growth factor, differentiation factors, anti-allergens, corticosteroids, stem cells, anti-adhesive molecules, an analgesic, an anti-inflammatory agent, an antibiotic, insulin, a hormone, a hyaluronic acid, mRNA or mRNA based drugs, cytotoxics, and vitamins.

Further, the active ingredient comprises or is at least one of: a siRNA based drug, verteporfin, genipin, antacids, antianxiety drugs, antiarrhythmics, antibacterials, coagulants, anticoagulants, thrombolytics, anticonvulsants, antidiarrheals, antiemetics, antifungals, antihistamines, antihypertensives, anti-inflammatories, antineoplastics, antipsychotics, antipyretics, antivirals, barbiturates, beta-blockers, bronchodilators, cold cures, cough suppressants, decongestants, diuretics, expectorants, hypoglycemics, immunosuppressives, laxatives, muscle relaxants, sedatives, sex hormones, sleeping drugs, and tranquilizers.

Preferably, the hydrogelating material has a degradation rate of 2 to 48 hours, 2 to 14 days, 2 to 8 weeks in vivo or 1 to 18 months in vivo. A desired degradation may depend on the application and can be tuned with the hydrogelating material. Example tuning methods for a degradation of the hydrogelating material are disclosed by Li, J., Mooney, D. Designing hydrogels for controlled drug delivery. Nat Rev Mater 1, 16071 (2016).

Preferably, the trigger is at least one of: a change in pH, a temperature, one or more enzymes, an electrical field, and mechanical stress, in particular shear stress.

Preferably, the trigger leads to a degradation of the hydrogelating material or the hydrogel.

Preferably, the felt and the hydrogelating material are directly connected to each other. E.g., the felt and the hydrogelating material may be felted to each other without using sutures or other additional attachment means such as clamps, etc. Additional attachment means may limit the area which can be felted reducing a distribution of forces.

Preferably, the connection is at least one of mechanical, chemical, and thermal.

Preferably, the fibers of the hydrogelating materials and the fibers of the felt are intertwined and/or entangled. The fibers of the hydrogelating material and the fibers of the felt may be felted to each other. A well distributed connection between the hydrogelating material and the fibers of the felt is established thereby. Further, this connection is particularly suitable for implanting the device using the felting techniques described in prior applications of the present applicant (e.g. in PCT/EP2024/055924). Further felting devices suitable for the disclosed device are disclosed in any of the following applications: PCT/CH2019/000015, PCT/EP2020/081887, PCT/EP2020/081891, and PCT/EP2020/081881 (all filed by the present applicant). In some examples, the needle may have a blunt or flat tip as e.g., described in EP 23159745.1.

Entangling and/or intertwining the fibers of the felt and the hydrogelating material with each other prevents unwanted interactions between the connection between the hydrogelating material and the felt and the felting needle during implantation of the device.

Preferably, the hydrogelating material comprises or is made of fibers having yield strength of at least 100, 250, or 500 kPa or at least 1, 10, or 100 MPa. Such fibers may be particularly advantageous as they may be compatible with felting, i.e. such fibers may be pushed or pulled by a felting needle.

Preferably, the hydrogelating material comprises or is made up of fibers having a tenacity between 0.5 and 9 cN/dtex. In particular, the fibers may have a tenacity of more than 1 cN/dtex.

Preferably, the patch comprising the felt (or a layer comprising felt) has at least one of: a thickness of 0.2 to 3 mm, particular 1.0 to 1.8 mm, an area weight of 10 to 500 g/m², in particular 200 to 400 g/m², a tensile strength of 50 to 1200 N, in particular 400 to 800 N and a stiffness of 10 to 500 N/mm², in particular 50 to 250 N/mm². Preferably, the felt comprises or is made up of fibers having a tenacity between 0.5 and 10 cN/dtex, in particular 3 and 9 cN/dtex. Such felts are particular suitable for being felted to soft tissue and allow for a good mechanical connection between the felt and soft tissue.

The hydrogelating material and the felt may be sutured together. This may provide for (additional) attachment.

Further, the hydrogelating material and the felt may be connected to each other by cross-linking the hydrogelating material within the felt. This may result in a composite (or combined) hydrogelating - felt material. Such a material is particularly useful since handling is improved and the felt can be attached directly to the soft tissue.

Preferably, the hydrogelating material and the felt are laminated to each other, in particular by heating opposing surfaces of the hydrogelating material and the felt material. This may provide for an alternative or additional mechanism of attachment between the hydrogelating material and the felt.

Preferably, the felt comprises or consists of fibers made of polyethylene terephthalate, PET, polylactic acid, PLA, polyglutamic acid, PGA hyaluronic acid, HA, polydioxanone, PDO, polylactic-co-glycolic acid PLGA, polytetrafluoroethylene, PTFE, polycaprolactone PCL, chitosan, poly-D-lactide, PDLA, poly-DL-lactide, PDLLA, or poly-L-lactide, PLLA.

Preferably, the patch includes a first layer comprising the felt. The first layer may include a plurality of holes and the hydrogelating material may be arranged within the holes. Thereby, a single layer may provide mechanical stability and a predetermined release profile for the active ingredient.

Preferably, the device includes a first layer comprising the felt and a second layer comprising the hydrogelating material. The layers may be stacked on top of each other. Thereby, a simple to manufacture and compact device with a predetermined active ingredient release profile is provided.

Preferably, the first layer comprises an edge region. The edge region may be folded around the second layer. In further examples, the device comprises multiple (e.g. 2, 3, 4) edge regions that are folded around the second layer. Thereby, the hydrogelating material may be kept in place and a release of the active ingredient may be retarded.

Preferably, the second layer additionally comprises a frame formed by a felt. The hydrogelating material may be held within the frame and may be surrounded by the frame.

Thereby, the hydrogelating material may be kept in place and a release of the active ingredient may be retarded. Further, handling of the device may be facilitated.

Preferably, the device comprises a third layer. The third layer may comprise a felt, e.g. the same felt or a different felt as the first layer. The third layer may additionally or alternatively comprise a hydrogelating material. The hydrogelating material may be the same hydrogelating material as in the second layer or a different hydrogelating material. The layers may be arranged in the following order: first layer (felt), second layer (hydrogelating material), third layer (felt). The layers may also be arranged in other orders: third layer (hydrogelating material), first layer (felt), second layer (hydrogelating material) or third layer (felt), first layer (felt), second layer (hydrogelating material).

Preferably, the second layer, i.e. the hydrogelating material, is sandwiched between two layers comprising the felt. Thereby, particularly slow-release profiles may be realized. Further, the hydrogelating material is secure and may be chosen from mechanically less stable hydrogelating materials.

Preferably, the third layer comprises a hydrogelating material and a pouch is between the second and third layers. A pouch may additionally enable storing active ingredients that are not held by water, e.g. active ingredients that are solid, gaseous, or held in a liquid other than water. Thereby, large amounts of active ingredients may be brought to a target site.

Preferably, the pouch comprises an active ingredient. The active ingredient may comprise a solid or a gas. The pouch may include a lining made of or comprising hydrogelating material. The hydrogelating material forming the pouch may be at least partially swollen forming a hydrogel. Swelling the hydrogelating material may prevent active ingredients having a solid or gaseous form from escaping the pouch.

Preferably, the device includes a first layer comprising the felt and a second layer comprising the hydrogelating material. The first layer may include a first surface and an opposing second surface. The second layer may comprise one or more stripes that traverse the first layer from the first surface to the opposing second surface and back. Thereby, the second layer may be interweaved within the first layer. This allows securing a large amount of second layers and thus of hydrogelating material.

Preferably, the device is configured for tendon repair, ligament repair, muscle repair, fascia repair, skin repair, hernia repair, gastro-intestinal surgery, cardiac surgery, vascular surgery, or dental surgery.

Preferably, the device is configured to seal internal wounds. In particular, the hydrogelating material is preferably configured to seal internal wounds. In particular, the hydrogelating material may be configured to seal a wound swollen, i.e. when forming a hydrogel. Thereby, a wound, e.g. a rupture, may be mechanically stabilized, treated with active ingredients and sealed at the same time.

Preferably, the internal wound is at least one of: a tendon rupture or tear, a ligament rupture or tear, an opening in an abdominal wall that seals the abdominal cavity or an opening in a wall of the gastrointestinal system.

A further aspect of the present invention relates to a set comprising a device as described above and a felting device. The felting device may be the felting device described in PCT/EP2024/055924.

The felting device may comprise a felting needle configured to push individual fibers into the soft tissue. The felting needle may be reciprocally movable between an extended position and a retracted position. In the extended position, a working tip of the felting needle protrudes beyond an aperture of the felting device. The aperture may be arranged at a distal end of the felting device. In the retracted position, the working tip of the felting needle does not protrude beyond the aperture. The extended position and the retracted position may be end positions of the reciprocal movement. The felting needle may be coupled to a driving mechanism with a force limiting element. During operation, the force limiting element may limit the force applied by the driving mechanism to the felting needle to a predetermined force threshold when the working tip is moved from the retracted position to the extended position.

A felting needle as mentioned herein may mean a needle that is configured to push individual (or at least only few) fibers into soft tissue. The felting needle may include barbs and/or a blunt tip that is configured to push the fibers (see e.g. EP 23159745.1).

When the felting needle moves towards the extended position and out of the aperture, the barbs or a tip of of the felting needle may catch individual fibers and drag the fibers into soft tissue. Then, the felting needle may be retracted again into the retracted position.

A reciprocal movement may be repeated with a frequency of 1-200 Hz, preferably 10-100 Hz, most preferably 20-80 Hz and in one embodiment 40 Hz. The reciprocal motion may have an amplitude of 1 to 25 mm, particularly preferred 2 to 20 mm, further preferred from 3 to 16 mm, most preferred from 4 to 10 mm. These amplitudes cover a sufficient thickness of feltable textiles while penetrating the target soft tissue at the same time and result in a sufficiently strong attachment between the feltable material and the target site.

A further aspect of the present invention relates to a method of manufacturing an implantable device for a soft tissue repair or closure. The implantable device may be the implantable device as described above. The method comprises the following steps:
a. Providing a felt patch and a hydrogelating material, wherein the felt patch is configured to be felted to soft tissue to repair or close the soft tissue and wherein the hydrogelating material is configured to retain an active ingredient and biodegradable with a predetermined degradation rate to release the active ingredient or wherein the hydrogelating material is configured to release the active ingredient in response to a trigger, and
b. Combining the felt patch and the hydrogelating material to each other.

Combining may mean attaching (e.g. mechanically, chemically, or thermally) as described above.

Preferably, attaching the felt patch and the hydrogelating material to each other comprises felting the felt patch and the hydrogelating material to each other, preferably using a felting device, e.g. a felting device as described above.

Preferably, the method comprises the step of loading the active ingredient in the hydrogelating material by hydrogelating the hydrogelating material to form an active ingredient loaded hydrogel. The hydrogelating material may be swollen with water. The active ingredient may be, e.g., dissolved, in the water.

The step of loading the active ingredient may be prior to or after an implantation of the device.

Preferred embodiments are disclosed by way of example only with respect to the following drawings:
Figure 1 shows a perspective view of a first example of a device according to the disclosure.
Figure 2 shows a perspective view of a second example of a device according to the disclosure.
Figure 3 shows a perspective view of a third example of a device according to the disclosure.
Figure 4 shows a side view and a perspective view of a fourth example of a device according to the disclosure.
Figure 5 shows a perspective view of a fifth example of a device according to the disclosure.
Figure 6 shows a perspective view of a sixth example of a device according to the disclosure.
Figure 7 shows a perspective view and a side view of a seventh example of a device according to the disclosure.
Figure 8 shows a perspective view of an eighth example of a device according to the disclosure.
Figure 9 shows a perspective view of a ninth example of a device according to the disclosure.
Figure 10 shows a perspective view and a side view of a tenth example of a device according to the disclosure.
Figure 11 shows a perspective view of an eleventh example of a device according to the disclosure.
Figure 12 shows a perspective view of a twelfth example of a device according to the disclosure.
Figure 13 shows a method of attaching the device shown in figure 1 to soft tissue.

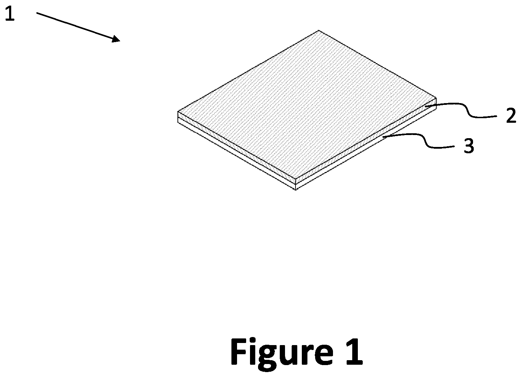

Figure 1 shows a device 1 according to the disclosure. The device 1 may be an implant. The device 1 comprises (or consists of) a first layer 2 and a second layer 3. The first layer 2 is made of a felt material. Any of the above-mentioned felt materials may be used. In this particular example, the felt material is made of PET fibers. The first layer 2 has a thickness of 1.8 mm and an area weight of 300 g/m². A length and a width of the first layer 2 may be adapted according to the required use. In one example, i.e. a rotator cuff tendon repair, the first layer may have a width of 25 mm and a length of 30 mm. The felt material is intended to be felted to soft tissue. The fibers of the felt have a tenacity of 6 cN/dtex. Tenacity (textile strength) is measured by dividing the breaking load of the fiber by its mass per unit length and may be expressed in N/tex or cN/dtex in the International System of Units. Tex is a mass per unit length of 1g/km. The fibers of the felt may be made of any of the materials mentioned above. In this example, the felt fibers in the first layer 2 have a fiber thickness of between 5 and 25 micrometers or 15 to 20 micrometers (e.g. with a tolerance of 5 micrometers). The fibers may have a fiber length of at least 40 or 50 mm (e.g. with a tolerance of 10 mm). In particular, the fiber length may be 50 to 60 mm.

The felt may be prepared as is known by bale opening, fiber carding, optionally web cross-lapping, pre-needling, finish-needling, optionally calendaring and winding up the nonwoven.

The second layer 3 is made of a hydrogelating material. The hydrogelating material may be any of the materials mentioned above. In this example, the hydrogelating material is carboxymethyl cellulose, CMC. The CMC is cross-linked and hydrophilic. The hydrogelating material can retain water. If the hydrogelating material retains water, it becomes a hydrogel. In the example, the second layer 3 may have a thickness of about 1 mm. In other examples the second layer 3 may have a thickness of 0.5 to 3 mm, in particular 0.5 to 2 mm.

Hydrogelating materials may be formed using a monomer, an initiator, and a cross-linker. To control heat of a polymerization and final hydrogels properties, diluents can be used, such as water or other aqueous solutions. Then, the hydrogel mass can be washed to remove impurities left from the preparation process. The impurities may include non-reacted monomers, initiators, cross-linkers, and unwanted products produced via side reactions.

Hydrogels may be precisely tuned to a particular purpose using various processing steps like polymerization. Example tuning mechanisms are disclosed by Li, J., Mooney, D. Designing hydrogels for controlled drug delivery. Nat Rev Mater 1, 16071 (2016). Further drug delivery mechanisms are disclosed by Cooper RC, Yang H. Hydrogel-based ocular drug delivery systems: Emerging fabrication strategies, applications, and bench-to-bedside manufacturing considerations. J Control Release. 2019 Jul 28;306:29-39.

The active ingredient, e.g. a drug, may be released using a trigger. As mentioned above, the trigger may be a change in pH, a temperature, one or more enzyme, and mechanical stress, in particular shear stress. Further, the trigger may be an electrical field as disclosed in Murdan S. Electro-responsive drug delivery from hydrogels. J Control Release. 2003 Sep 19;92(1-2):1-17. doi: 10.1016/s0168-3659(03)00303-1. PMID: 14499181.

In one particular example the CMC is loaded with a liquid non-steroidal anti-inflammatory drug (NSAID, e.g. ibuprofen). The NSAID will then be released in a controlled manner by degradation of the second layer 3 of the device.

Herein, the second layer 3 may also be called a responsive layer, as the second layer may release an active ingredient in response to a trigger as described herein. Accordingly the first layer 2 may be described as a non-responsive layer.

The second layer 3 may have a similar thickness as the first layer 2. The thickness may also be tuned depending on a desired loading of the active ingredient, expected mechanical loads and the available space for the application. The second layer 3 may also have a same width and length as the first layer 2. In some examples, the second layer 3 may have a smaller width and/or length as the first layer. The first layer 2 may include an edge region that is not covered by the second layer and may be directly attached to soft tissue.

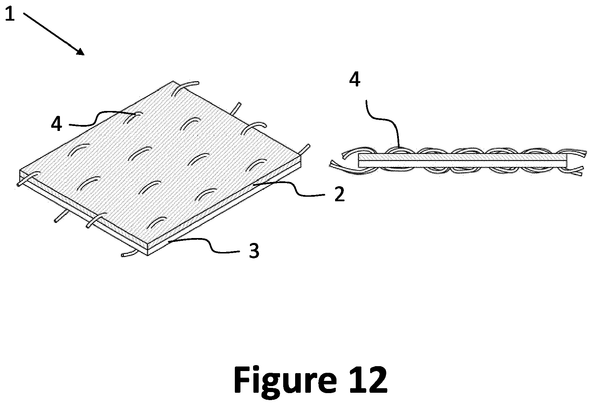

As mentioned above, the first layer 2 and the second layer 3 may be connected using sutures 4 (see figure 12) or may be laminated together.

Preferably, the first layer 2 and the second layer are felted together. The hydrogelating material may include fibers having a tenacity of at least 0.5 cN/dtex. An example fiber made of natural cellulose fibers having a tenacity of 2.9 g/den (about 2.6 cN/dtex) is disclosed in Narendra Reddy, Yiqi Yang, Properties and potential applications of natural cellulose fibers from the bark of cotton stalks, Bioresource Technology, Volume 100, Issue 14, 2009, Pages 3563-3569, ISSN 0960-8524. Such fibers may also be used in hydrogelating materials. Another example hydrogelating material having tenacity fibers and suitable for drug loading is disclosed in Kim GO, et al. An electrostatically crosslinked chitosan hydrogel as a drug carrier. Molecules 2012; 17: 13704-13711.

Further fibers for hydrogelating materials are disclosed in Lam NYK, Zhang M, Guo H, Ho CP, Li L. Effect of fiber length and blending method on the tensile properties of ring spun chitosan-cotton blend yarns. Textile Research Journal. 2017;87(2):244-257.

Hydrogelating materials with fibers having a high tenacity are particularly advantageous, as they may be felted to the patch comprising a felt for tissue repair. Through felting, both types of fibers (of the first layer 2 and the second layer 3) are intertwined and entangled. This creates a sufficient connection between the first layer 2 and the second layer 3. In some examples, the first layer 2 and the second layer 3 may be felted together during manufacture with known textile manufacturing methods. In other examples, the first layer 2 and the second layer 3 may be manufactured and delivered as parts (e.g. as a set). Then, the first layer 2 and the second layer 3 may be connected by a user, e.g. using a felting device.

An example felting device for connecting the first layer 2 and the second layer 3 is disclosed in PCT/EP2024/055924. The first layer 2 and the second layer 3 may be laid on top of each other and felted to each other using the moving needle disclosed in PCT/EP2024/055924.

In an example manufacturing method, the first layer 2 and the second layer 3 are positioned on top of each other. Both may be positioned on an appropriate substrate through which a felting needle can penetrate (e.g., a synthetic sponge or foam material). The layers may be felted with the device disclosed in PCT/EP2024/055924 any other felting device. For example, the layers may be hand-felted (or machine felted) with an array of (more than 2, e.g. 6) felting needles. The felting needles may have a 42 gauge and barbs. The felting needles may have at least a 36 gauge. The barbs may have a close spacing.

First, the device 2 be felted from side of the first layer 2, pushing fibers of the first layer into the second layer. Then, the device may also be felted from the side of the second layer 3, pushing fibers of the second layer into the first layer. A felting depth of the needle may be 10 mm or less and a punch density (i.e. punches per area) may be in a range between 20 to 40 needle punches / cm².

In this manner, the hydrogelating CMC (second layer 3) and the first layer 2 made of PET may be connected to each other, providing a feltable device.

In some examples, the hydrogelating material may be loaded with the active ingredient prior to introduction of the device into the human body. Because of the expected presence of body fluids in most clinical applications, loading of the active ingredient should happen before implantation to avoid that the hydrogelating material soaks up body fluids instead of a composition comprising the active ingredient. Nevertheless, nonliquid active ingredients may still be added after implantation.

Loading the active ingredient may involve soaking the hydrogelating material with water forming a hydrogel layer. In an example process, an active ingredient is dissolved in water. Then, an active ingredient is loaded into the device by hydrogelating the hydrogelating material of second layer 3. In particular, the device 1 shown in figure 1 may be swollen with a liquid NSAID (e.g. ibuprofen) by injecting the drug into the responsive CMC layer (e.g. in the operating room). The hydrogelation starts and the responsive layer of the patch turns into the gelatinous state containing the drug in its crosslinked molecular chambers. Then, the device may be implanted covering an internal wound via felting using a surgical felting device as e.g. described in PCT/EP2024/055924. A needle of the surgical felting device anchors fibers of the first layer 2 and the second layer 3 in the soft tissue around the wound and generates a strong reinforcement of the tissue. The second layer 3 may be arranged between the attachment surface of the soft tissue and the first layer 2. The second layer 3 with its direct contact to the ruptured soft tissue may additionally seal the wound and may slowly degrade due to the bodily fluids. At the same time, the degradation may cause the hydrogel to release the previously loaded NSAID and helps to avoid inflammations and pain to the patient. If the second layer 3 is fully degraded, the non-responsive PET nonwoven will keep up the mechanical strength to ensure avoidance of re-rupturing the open wound.

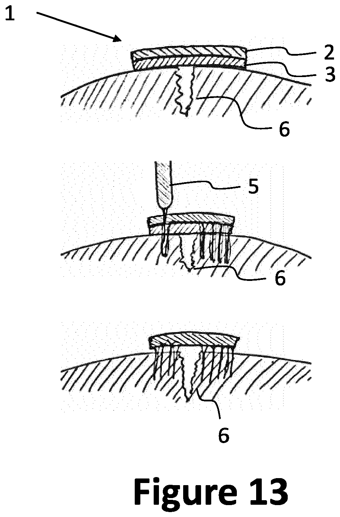

An example implantation process is illustrated in figure 13. Figure 13 shows in the first depiction (labelled A), the device 1 (or more precisely a two-layered patch) according to figure 1. The device 1 is positioned across a rupture 6 in soft tissue. A second depiction (labelled B) shows the process of surgical felting with a surgical felting device 5 as described in PCT/EP2024/055924, anchoring fibers of both PET and CMC into the soft tissue around the wound. A third depiction (labelled C) shows the rupture after degradation of the second layer 3, where the rupture experienced some healing (e.g. decreased in size). The nondegradable PET layer ensures the mechanical strength of the device. All shown example devices may be implanted in this manner. In some examples the first layer 2 may also be degradable.

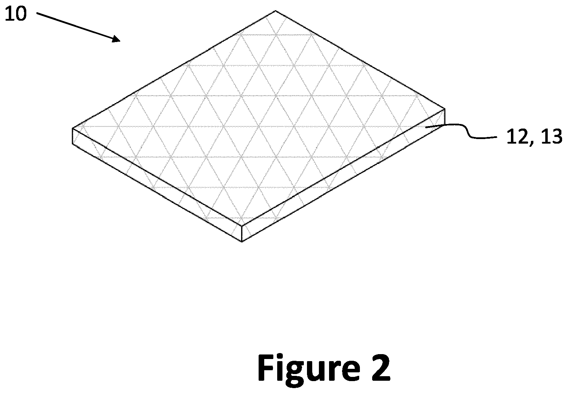

An example second device 10 is shown in figure 2. Similarly to the first device 1, the second device 10 includes a first layer 12 and a second layer 13. These are manufactured similarly as the first layer 2 and the second layer 13. However, instead of manufacturing the hydrogelating material for the second layer 13 and the felt for the first layer 12 separately, the hydrogelating material is crosslinked within the felt of the first layer. For example, the first layer is provided and at least partially, preferably fully, immersed in a solution including the monomers, an initiator, and a cross-linker. Then, cross-linking of the hydrogel may occur within the first layer 12 resulting in a blended layer including both, the hydrogelating material and the felt. Thereby, a combined felt and hydrogelating layer is formed.

In a further example, the second device 2 having a combined felt and hydrogelating layer may be formed by providing to layers separately as described with reference to figure 1. The fibers may be mechanically blended. For example, the fibers of the first layer and the second layer may be carded together and then felted forming a single layer. This may provide a mechanical blend. Thus, the fibers of the combined felt and hydrogelating layer would be entangled without necessarily being chemically bonded.

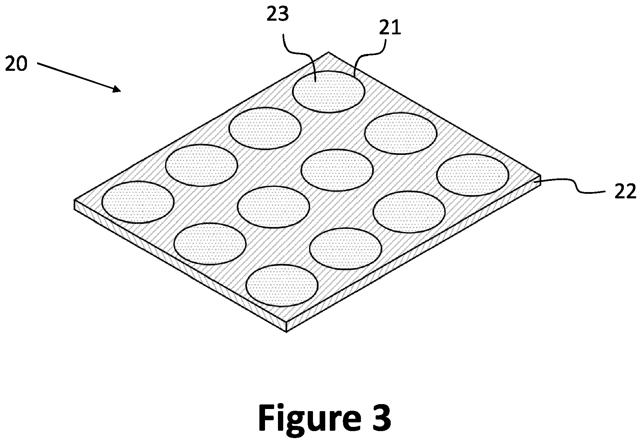

A third example device 20 is shown in figure 3. Figure 3 shows a device 20 with a first layer 22 that is similar to first layer 2. The first layer 22 includes multiple holes 21 that may or may not be arranged along a grid pattern as shown in figure 3. Each of the holes may have a width or diameter of 1 mm or more. The holes 21 may be formed by cutting, punching or otherwise. The holes 31 may be filed with a hydrogelating material 23. As described above, the hydrogelating material 23 may be loaded with an active ingredient by hydrogelating the hydrogelating material 23 to a hydrogel.

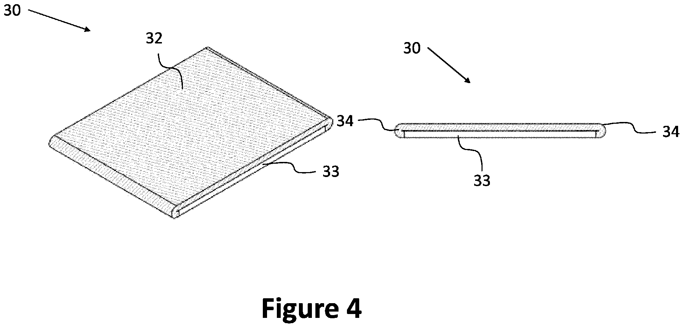

A fourth example device 30 is shown in figure 4. The device 30 is similar to the device 1 shown in figure 1 and includes a first layer 32 and a second layer 33. In addition, one or more edges 34 of the first layer 32 may be folded around and fixated, e.g. felted, in the folded position. In one example, as shown in figure 4, two opposing edges may be folded. In another example, all four edges of the first layer 32 may be folded and fixated (e.g. by felting). This may create a nest for the second layer 33 and may help keeping the second layer 33 in place after hydrogelation.

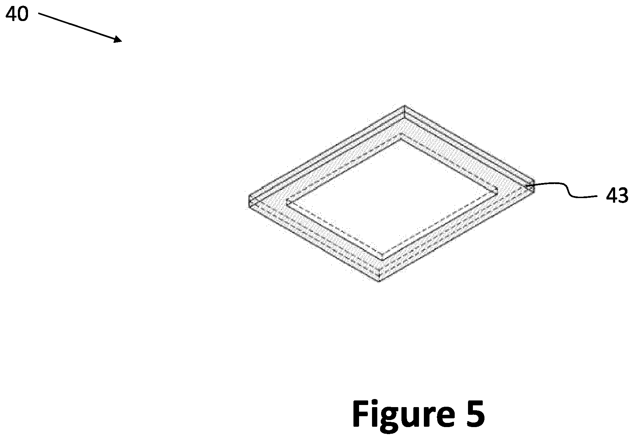

A portion of a fifth example device 40 is shown in figure 5. The device 40 includes a third layer 43. The third layer 43 forms a frame as shown in figure 5. This frame may surround the second layer 3 of the device shown in figure 1. Accordingly, the second layer 3 may be held within the third layer 43. As shown in figure 1, the first layer 2 is attached to the second layer which may be framed by the third layer 43. This arrangement may have a superior mechanical strength due to the frame. Further, when the third layer 43 surrounds the responsive second layer 3, a slower degradation of the hydrogel may occur.

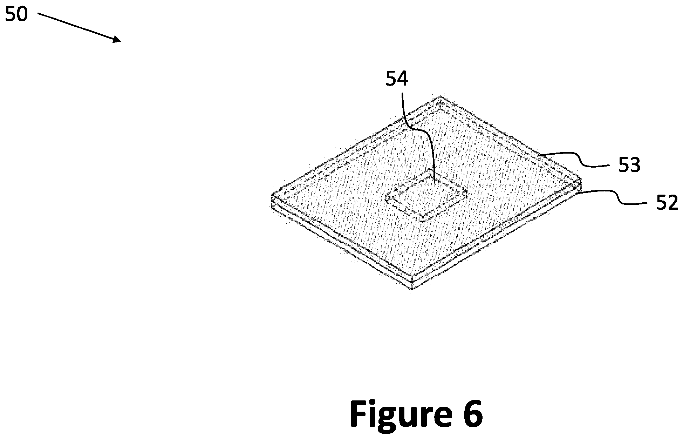

A sixth example device 50 is shown in figure 6. The device 50 includes a first layer 52, and a second layer 53. The second layer 53 comprises a pillar 54. The pillar 54 may be made of a non-responsive material, e.g. a felt material as described above, and is attached to the first layer 52. Further the second layer 53 may comprise the hydrogelating material. In the example, the remainder of the second layer 53 may be similar to the second layers described above and may surround the pillar 54. Thereby, a mechanically strong anchor in the center of the device is provided. Such an implant may be advantageous for torn tendon repair.

The described examples may be combined in any way. For example, the combined felt and hydrogelating layer may replace the first layer in any of the shown examples. In another example, the combined felt and hydrogelating layer may replace the second layer of hydrogelating material in any of the shown examples.

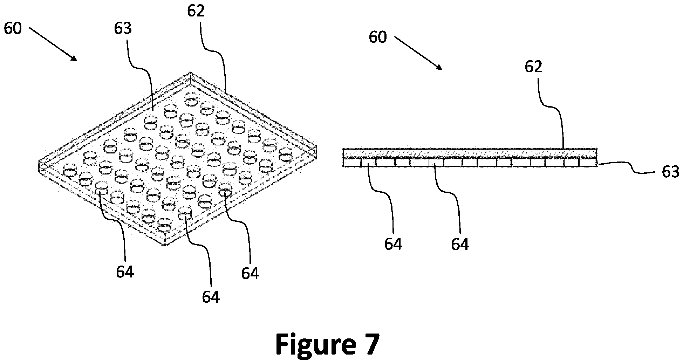

A seventh example device 60 is shown in figure 7. The device 60 includes a first layer 62 and a second layer 63 that are similar to first layer 2 and second layer 3. In addition, the second layer 63 includes a set of holes 64. These holes 64 may be distributed along a grid as shown in figure 7. The holes 64 may also be distributed in any other fashion. The device 60 may include one or more markers on an opposing side indicating the position of holes 64. Therefore, when using a felting method as implantation procedure onto soft tissue, more fibers of the first layer 62 are carried into the soft tissue which may increase the mechanical attachment of the patch to the soft tissue.

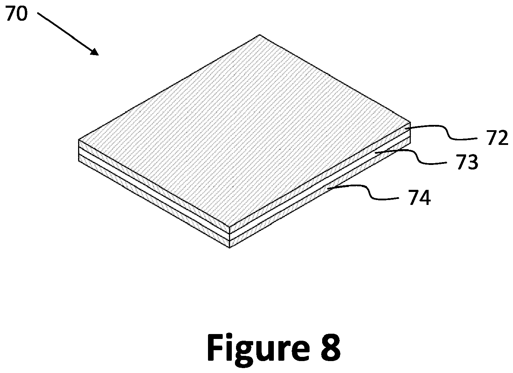

Further examples may include three or more layers. For example, as shown in figure 8 (device 70), a second layer 73 (similar to second layer 3) may be arranged between a first layer 72 (similar to first layer 2) and a third layer 74. The third layer 74 may be similar to the first layer 72. For example, the third layer 74 may be made of a felt material having similar fibers or different fibers. In particular, the third layer 74 is non-responsive. Such a device is mechanically particularly stable and leads to a slower release of the active ingredient. In a further variation, the first layer 72 may be sandwiched between two layers of hydrogelating material, e.g. between two second layers 73. Such a device may be advantageous if different active ingredients are to be released from the opposing sides of the device 70.

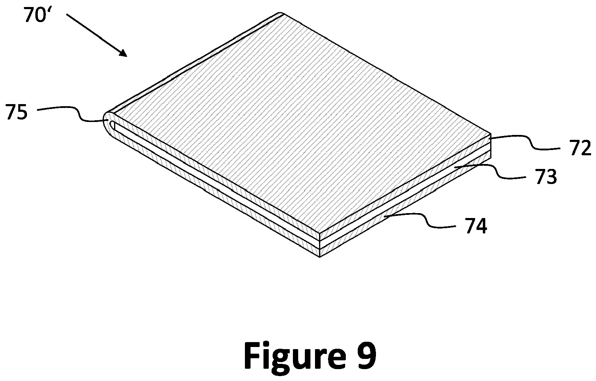

Figure 9 shows an alternative three-layered device 70. Instead of providing the first layer 72 and third layer 74 as separate sheets, a single sheet 75 of felt may be folded around the hydrogelating material (second layer 73).

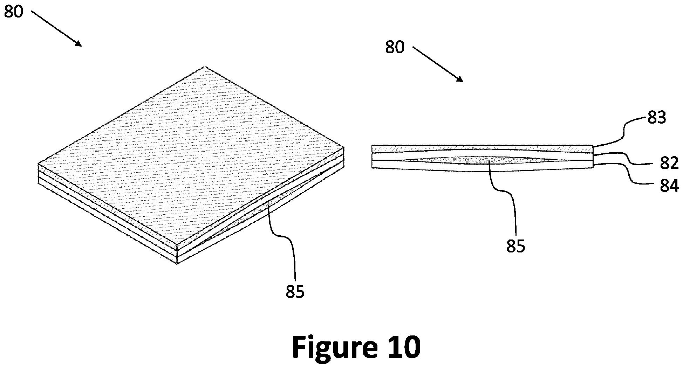

A tenth example device 80 is shown in figure 10. The device 80 includes a first layer 82 (similar to first layer 2) and a second layer 83 (similar to second layer 3). In addition, a third layer 84 is (e.g., directly) attached to the first layer 82. The third layer 84 may be similar to the first layer 82. For example, the third layer 84 may be made of a felt material having similar fibers or different fibers. In particular, the third layer 84 is responsive. The third layer 84 and the first layer 82 may form a pouch 85. The pouch 85 may be filled with hydrogelating material as described above. The pouch 85 does not need to be filled with a hydrogelating material. Instead or additionally, the pouch could contain liquids, solids or gases as one or more active ingredients that are not held in hydrogelating material. The pouch 85 may include an outer casing of hydrogelating material or hydrogel to seal the pouch 85. Swelling of the hydrogelating material forming the outer casing may seal the pouch 85. Thereby, large amounts of active ingredients may be released after the outer casing has degraded.

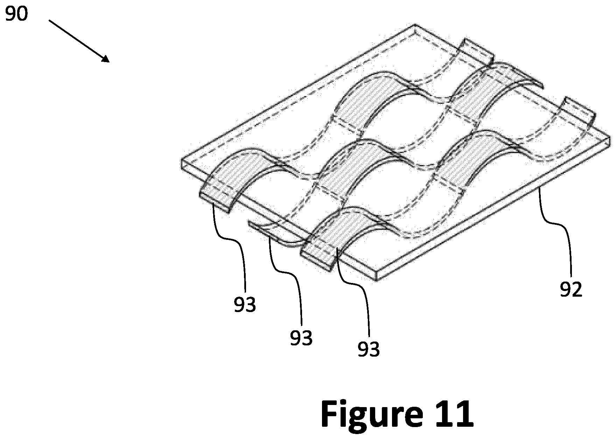

An eleventh example device 90 is shown in figure 11. The device 90 includes a first layer 92 (similar to first layer 2). Further, device 90 includes several second layers 93. Each of the second layers 93 may be made of similar materials as second layer 3. Preferably, one or more of the second layers 93 are formed by the combined layers disclosed in connection with figure 2. One or more of the second layers 93 may extend longitudinally and may have the shape of stripes as shown in figure 11. These stripes may be interwoven with the first layer 92. In one example, the stripes may be interwoven in an expected tensile direction of the device 90. For example, if the device 90 is used to bridge a rupture as shown in figure 13, then the tensile direction may extend across the rupture. Thereby, the responsive layer (second layers 93) becomes large and can carry large amounts of active ingredients, whereas the second layer 93 (non-responsive layer) of nonwoven (or felt) keeps the device in place and provides mechanical strength. The stripes could also be interweaved in multiple directions crossing each other, forming a web-like structure.

One example procedure of using the disclosed devices may include the following steps.
1. Load the active ingredient (e.g., dissolved in water) to one of the second layers and/or one of the hydrogelating materials, which may consequently hydrogelate.
2. Deliver the device to a target area.
3. Attach the device using the surgical felting device as e.g. described with reference to figure 13.
4. Degradation of hydrogel causes precise release of the active ingredient.

Another example procedure of using the disclosed devices may include the following steps:
1. Deliver the device to a target area.
2. Attach the device using the surgical felting device as e.g. described with reference to figure 13.
3. Load the active ingredient to one of the second layers and/or one of the hydrogelating materials.
4. Degradation of hydrogel causes precise release of the active ingredient.

Further aspects of the present disclosure relate to the following
1. Implantable medical device (1) for a tissue repair or closure, wherein the device comprises:
   a. a patch (2) comprising a felt for the tissue repair or closure, and
   b. a hydrogelating material (3) configured to retain an active ingredient, wherein the hydrogelating material is biodegradable with a predetermined degradation rate to release the active ingredient or wherein the hydrogelating material is configured to release the active ingredient in response to a trigger.
2. Device according to aspect 1, wherein the felt is configured to be felted.
3. Device according to aspect 1 or 2, wherein the hydrogelating material comprises or is made of a natural polymer or a synthetic polymer or a combination thereof.
4. Device according to aspect 3, wherein the natural polymer comprises or is at least one of: cellulose, in particular bacterial cellulose or carboxymethyl cellulose, CMC, alginate, hyaluronic acid, HA, gelatine, agar, agarose, starch, chitosan, and fibrinogen.
5. Device according to aspect 3, wherein the synthetic polymer is at least one of: an acrylamide, an acrylic acid, a salt of an acrylic acid, and 2-Hydroxyethyl Methacrylate, PHEMA.
6. Device according to one of the preceding aspects, wherein the hydrogelating material forms a hydrogel and is partially swollen with water.
7. Device according to one of the preceding aspects, wherein the hydrogelating material forms a hydrogel and wherein the hydrogel is fully swollen.
8. Device according to one of the two preceding aspects, wherein the hydrogel comprises a active ingredient.
9. Device according to the preceding aspect, wherein the active ingredient is water-based and wherein the active ingredient is in particular dissolved in the water held by the hydrogel.
10. Device according to one of the two preceding aspects, wherein the active ingredient is dispersed, suspended, and/or emulsified in water held by the hydrogel.
11. Device according to one of the three preceding aspects, wherein the active ingredient comprises at least one of: a growth factor, a differentiation factor, an anti-allergen, a corticosteroid, stem cells, anti-adhesive molecules, an analgesic, an anti-inflammatory agent, an antibiotic, insulin, a hormone, hyaluronic acid, mRNA or mRNA based drugs, cytotoxics, and vitamins.
12. Device according to one of the preceding aspects, wherein the active ingredient is at least one of: a drug, a bioactive component and a biologic.
13. Device according to one of the preceding aspects, wherein the hydrogelating material has a degradation rate of 2 to 48 hours, 2 to 14 days, 2 to 8 weeks in vivo or 1 to 18 months in vivo.
14. Device according to one of the preceding aspects, wherein the trigger is at least one of: a change in pH, a temperature, one or more enzyme, an electrical field, and mechanical stress, in particular shear stress.
15. Device according to one of the preceding aspects, wherein the trigger leads to a degradation of the hydrogelating material.
16. Device according to one of the preceding aspects, wherein the felt and the hydrogelating material are directly connected to each other.
17. Device according to the preceding aspect, wherein the connection is at least one of mechanical, chemical, and thermal.
18. Device according to one of the preceding aspects, wherein fibers of the hydrogelating material and fibers of the felt are intertwined and/or entangled, in particular felted to each other.
19. Device according to one of the preceding aspects, wherein the hydrogelating material comprises or is made up of fibers having a yield strength of at least 100, 250, or 500 kPa or at least 1, 10, or 100.
20. Device according to one of the preceding aspects, wherein the hydrogelating material comprises or is made up of fibers having a tenacity between 0.5 and 9 cN/dtex, in particular more than 1 cN/dtex.
21. Device according to one of the preceding aspects, wherein the felt has at least one of: a thickness of 1.0 to 1.8 mm, an area weight of 200 to 400 g/m², a tensile strength of 400 to 800 N and a stiffness of 50 to 250 N/mm².
22. Device according to one of the preceding aspects, wherein the felt comprises or is made up of fibers having a tenacity between 3 and 9 cN/dtex.
23. Device according to one of the preceding aspects, wherein the hydrogelating material and the felt are sutured together.
24. Device according to one of the preceding aspects, wherein the hydrogelating material and the felt are connected to each other by cross-linking the hydrogelating material within the felt, forming a composite hydrogelating-felt material.
25. Device according to one of the preceding aspects, wherein the hydrogelating material and the felt are laminated to each other.
26. Device according to one of the preceding aspects, wherein the felt comprises or consists of fibers made of polyethylene terephthalate, PET, polylactic acid, PLA, polyglutamic acid, PGA, , polydioxanone, PDO, polylactic-co-glycolic acid PLGA, polytetrafluoroethylene, PTFE, polycaprolactone PCL, chitosan, poly-D-lactide, PDLA, poly-DL-lactide, PDLLA, or poly-L-lactide, PLLA.
27. Device according to one of the preceding aspects, wherein the patch includes a first layer comprising the felt, wherein the first layer includes a plurality of holes and wherein the hydrogelating material is arranged within the holes.
28. Device according to one of the preceding aspects, including a first layer comprising the felt and a second layer comprising the hydrogelating material.
29. Device according to the preceding aspect, wherein the first layer comprises an edge region and wherein the edge region is folded around the second layer.
30. Device according to one of the two preceding aspects wherein the second layer additionally comprises a frame formed by a felt, wherein the hydrogelating material is held within and surrounded by the frame.
31. Device according to one of the three preceding aspects comprising a third layer,
   wherein the third layer comprises the felt and/or the hydrogelating material.
32. Device according to the preceding aspect, wherein the second layer is sandwiched between two layers comprising the felt.
33. Device according to one of the two preceding aspects, wherein the third layer comprises a hydrogelating material and wherein a pouch is between the second and third layers.
34. Device according to the preceding aspect, wherein the pouch comprises an active ingredient, the active ingredient preferably comprising a solid or a gas, wherein the hydrogelating material forming the pouch is preferably at least partially swollen forming a hydrogel.
35. Device according to one of the preceding aspects, wherein the device includes a first layer comprising the felt, and a second layer comprising the hydrogelating material, wherein the first layer includes a first surface and an opposing second surface and wherein the second layer comprises one or more stripes that traverse the first layer from the first surface to the second surface and back.
36. Device according to one of the preceding aspects, wherein the device is configured for tendon repair, ligament repair, hernia repair, gastro-intestinal surgery, or dental surgery.
37. Device according to one of the preceding aspects, wherein the device, in particular the hydrogelating material, is configured to seal internal wounds.
38. Device according to one of the preceding aspects, wherein the internal wound is at least one of: a tendon or ligament tear, an opening in an abdominal wall sealing an abdominal cavity, or an opening in a wall of the gastrointestinal system.
39. Set comprising a device according to one of the preceding aspects and a felting device.
40. Set according to the previous aspect, wherein the felting device comprises a felting needle configured to push individual fibers into the soft tissue.
41. Set according to the previous aspect, wherein the felting needle is reciprocally movable between an extended position in which a working tip of the felting needle protrudes beyond an aperture of the felting device arranged at a distal end of the felting device and a retracted position in which the working tip of the felting needle does not protrude beyond the aperture.
42. Set according to the previous aspect, wherein the extended position and the retracted position are end positions of the reciprocal movement.
43. Set according to one of the three preceding aspects wherein the felting needle is coupled to a driving mechanism via a force limiting element which during operation limits the force applied by the driving mechanism to the felting needle to a predetermined force threshold when the working tip is moved from the retracted position to the extended position.
44. Method of manufacturing an implantable device for a soft tissue repair or closure, preferably a device according to one of the preceding aspects, comprising the steps of:
   a. Providing a felt patch and a hydrogelating material, wherein the felt patch is configured to be felted to soft tissue to repair or close the soft tissue and wherein the hydrogelating material is configured to retain an active ingredient and biodegradable with a predetermined degradation rate to release the active ingredient or wherein the hydrogelating material is configured to release the active ingredient in response to a trigger, and
   b. Combining the felt patch and the hydrogelating material to each other.
45. Method according to the preceding aspect, wherein attaching the felt patch and the hydrogelating material to each other comprises felting the felt patch and the hydrogelating material to each other.
46. Method according to one of the three preceding aspects, comprising the step of loading the active ingredient in the hydrogelating material by hydrogelating the hydrogelating material to form a active ingredient-loaded hydrogel.
47. Method according to the preceding aspect, wherein loading the active ingredient comprises providing a water-based active ingredient and hydrogelating the hydrogelating material to form the active ingredient-loaded hydrogel.
48. Method according to one of the four preceding aspects, wherein the step of loading a active ingredient is prior to or after an implantation of the device.

## Claims

1. Implantable medical device (1) for a tissue repair or closure, wherein the device comprises:
a. a patch (2) comprising a felt for the tissue repair or closure, and
b. a hydrogelating material (3) configured to retain an active ingredient, wherein the hydrogelating material is biodegradable with a predetermined degradation rate to release the active ingredient or wherein the hydrogelating material is configured to release the active ingredient in response to a trigger.

2. Device according to claim 1, wherein the felt is configured to be felted.

3. Device according to one of the preceding claims, wherein the hydrogelating material forms a hydrogel and is partially or fully swollen with water and comprises the active ingredient.

4. Device according to one of the preceding claims, wherein the active ingredient is water-based and wherein the active ingredient is in particular dissolved in the water held by the hydrogel.

5. Device according to one of the preceding claims, wherein the trigger leads to a degradation of the hydrogelating material.

6. Device according to one of the preceding claims, wherein fibers of the hydrogelating material and fibers of the felt are intertwined and/or entangled, in particular felted to each other.

7. Device according to one of the preceding claims, wherein the hydrogelating material comprises or is made up of fibers having a yield strength of at least 100, 250, or 500 kPa or at least 1, 10, or 100 and/or the hydrogelating material comprises or is made up of fibers having a tenacity between 0.5 and 9 cN/dtex, in particular more than 1 cN/dtex.

8. Device according to one of the preceding claims, wherein the hydrogelating material and the felt are connected to each other by cross-linking the hydrogelating material within the felt, forming a composite hydrogelating-felt material.

9. Device according to one of the preceding claims, wherein the patch includes a first layer comprising the felt, wherein the first layer includes a plurality of holes and wherein the hydrogelating material is arranged within the holes.

10. Device according to one of the preceding claims, including a first layer comprising the felt and a second layer comprising the hydrogelating material.

11. Device according to the preceding claim, wherein the second layer is sandwiched between two layers comprising the felt.

12. Device according to one of the preceding claims, wherein the device is configured for tendon repair, ligament repair, muscle repair, fascia repair, skin repair, hernia repair, gastro-intestinal surgery, cardiac surgery, vascular surgery, or dental surgery.

13. Set comprising a device according to one of the preceding claims and a felting device.

14. Set according to the previous claim, wherein the felting device comprises a felting needle configured to push individual fibers into the soft tissue.

15. Method of manufacturing an implantable device for a soft tissue repair or closure, preferably a device according to one of claims 1 to 12, comprising the steps of:
a. Providing a felt patch and a hydrogelating material, wherein the felt patch is configured to be felted to soft tissue to repair or close the soft tissue and wherein the hydrogelating material is configured to retain an active ingredient and biodegradable with a predetermined degradation rate to release the active ingredient or wherein the hydrogelating material is configured to release the active ingredient in response to a trigger, and
b. Combining the felt patch and the hydrogelating material to each other.
